# EUROPEAN PATENT SPECIFICATION

(11) **EP 2 403 436 B1**
(45) Date of publication and mention of the grant of the patent: **28.04.2021**
(21) Application number: 09827813.8
(22) Date of filing: 24.11.2009
(51) Int. Cl.: A61F 2/38

(54) **FOAM CUSHION FOR INSERTION IN A KNEE JOINT**
SCHAUMSTOFFPOLSTER ZUR EINFÜHRUNG IN EIN KNIEGELENK
COUSSIN DE MOUSSE À INSÉRER DANS UNE ARTICULATION DU GENOU

(30) Priority: 24.11.2008 US 193396 P
(43) Date of publication of application: 11.01.2012
(73) Proprietor: Implantica Patent Ltd., 223 70 Lund (SE)
(72) Inventor: FORSELL, Peter, 6300 Zug (SE)
(86) International application number: PCT/SE2009/000502
(87) International publication number: WO 2010/059098

(56) References cited:
- FR-A1- 2 693 650
- FR-A1- 2 693 650
- US-A- 5 344 459
- US-A- 5 344 459
- US-A1- 2004 236 424
- US-A1- 2006 190 078
- US-B1- 6 530 956

## Description

The present application relates to knee joint repairs, and more particularly, to a cushion for insertion in a patient's knee joint that acts as a buffer between the patient's upper and lower knee bones.

### BACKGROUND OF THE INVENTION

Most people experience some kind of knee problem at some time during their life. Many knee injuries occur during physically stressful activities, such as sports. It is possible, however, for knee problems to develop from everyday wear and tear or overuse of a person's knees.

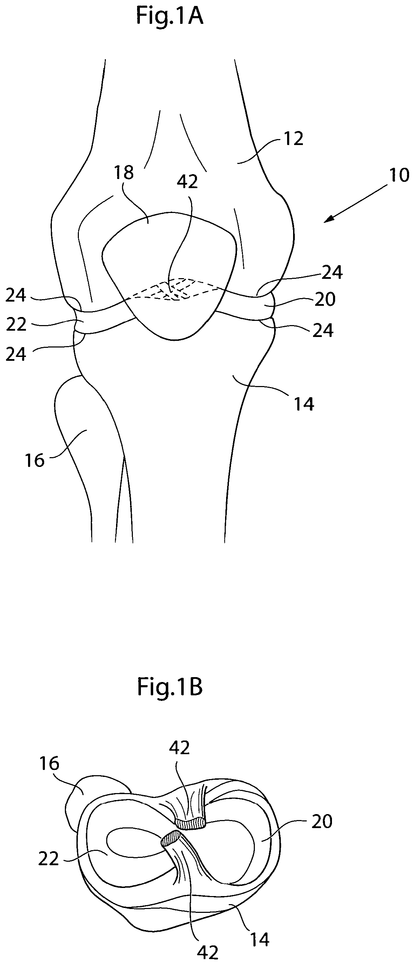

FIGURE 1A is a front view of a healthy knee joint 10. The knee joint 10 includes an upper bone called the femur or "thigh bone" 12, two lower bones called the tibia or "shin bone" 14 and the fibula 16, and a patella or "knee cap" 18. The upper and lower bones 12 and 14 are separated by two rubbery cushions or discs which are called the "medial meniscus" 20 and the "lateral meniscus" 22, which are best seen in the top view of FIGURE 1B. The upper and lower bones are also connected by ligaments, tendons and muscles (not shown). The surfaces of the upper and lower bones inside the knee joint 10 are covered by a layer of smooth, shiny cartilage called "articular cartilage" 24, which protects these bones, absorbs shock and provides a smooth, gliding surface for near frictionless movement of the bones as the knee joint 10 is flexed, bent or extended.

Sudden or acute injuries are the most common cause of knee problems. One kind of acute injury is one or more tears occurring in one or both of the menisci 20 and 22 that serve to cushion the knee joint 10.

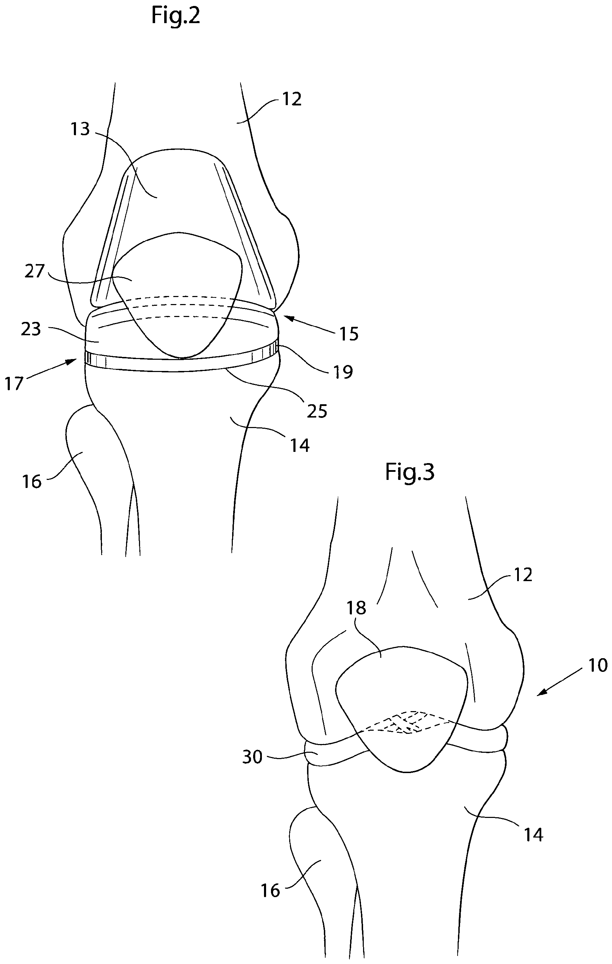

When a person's problems with a knee joint become severe enough, he or she may require a total knee replacement. FIGURE 2 is a front view of a conventional total knee replacement, or "arthroplasty" 11 in which the end surfaces of the upper and lower knee bones 12 and 14 are relined with artificial parts or "prostheses". Typically, there are three components used in a conventional knee replacement. The femoral (thigh) component 13 is made of metal and is inserted into the end 15 of the femur 12. The tibia (shin bone) component 17, is made of a metal piece 19 that covers the end 21 of the tibia 14 and a polyethylene (medical-grade plastic) piece 23, which together cover the top end 25 of the tibia 14. The metal piece 19 forms the base of the tibia component 17, while the polyethylene piece 23 is attached to the top of the metal piece 19 to serve as a cushion and smooth gliding surface for the metal femoral component 13. The third component, the patella (kneecap) 27 may be all polyethylene or a combination of metal and polyethylene. These new knee replacement components are then stabilized by a patient's ligaments and muscles, just as in a healthy knee.

One draw back to the conventional knee replacement shown in FIGURE 2 is the wear and tear in the knee replacement that can result from the metal femoral component 13 rubbing against the polyethylene piece 23 of the tibia component 17. As such, it would be desirable to provide a solution to knee joint injuries, like tears in one or both of; the menisci 20 and 22 that cushion the upper and lower knee bones, that eliminates the wear and tear in a conventional knee replacement resulting from the metal femoral component 13 rubbing on the polyethylene piece 23 of the tibia component 17. Prior art US 2006/0190078 (Fell Barry) discloses a method and system for repair of a defect area in a joint by supplying a mold and inject material therein. Document US5 344 459 (Swartz Stephen) discloses an inflatable prosthesis which is implantable arthroscopically comprising a ring or a pair of annularly-shaped rings joined to each other laterally. FR 2 683 650 (Simon Henri) discloses atotal hip replacement prosthesis, including a cavity and some deformable material.

### BRIEF DESCRIPTION OF THE INVENTION

The present invention relates to a cushion adapted to be implanted in a patient's knee joint, between the femur and tibia bone. The cushion comprises a pocket made from a flexible material, and a fluid filling the interior of the pocket. The pocket is sized to fit in the space between the patient's femur and tibia bones. Further, the cushion comprises an internal control unit adapted to be implanted and to control the pressure in the implanted cushion, non-invasively when implanted. The cushion, when implanted, is adapted to allow pressure to be added to or removed from the fluid filling the cushion non-invasively. Further, the cushion comprises a pressure sensor connected to the cushion that, postoperatively when implanted, determines the level of pressure in the cushion, and is connected to the implanted control unit. Thus, the cushion serve as a buffer between the femur and tibia. The cushion is filled with a substantially incompressible fluid, which is capable of supporting the entire weight of the patient's body. The cushion is preferably made from a flexible material that allows relative movement between the patient's femur and tibia, while resisting rupture as it supports these two bones as they are moving relative to one another as the knee joint is flexed, bent or extended. Preferably, the cushion is made from a polymer type of material.

The cushion has a single interior pocket or compartment which is filled by the substantially incompressible fluid. The cushion can also be formed with a plurality of individual interior pockets or compartments within the overall cushion pocket which are filled by the substantially incompressible fluid. The plurality of pockets or compartments allows the integrity of the cushion to remain largely intact so that the cushion can continue to buffer the upper and lower bones of a patient's knee, even where one of the cushion's compartments ruptures for one reason or another. The plurality of pockets or compartments also provides, if there is extra pressure in one area, the ability to move fluid away from that area.

The cushion can also include an opening or hole in its center to accommodate the two interior crucible ligaments extending between the bottom of the femur and the top of the tibia. Alternatively, the cushion can be shaped like a bow tie with a narrow center portion that can fit between the two interior crucible ligaments extending between the femur and tibia. Any other suitable shape may be used, depending on the situation in a patient's knee, such as for example where there are no crucial ligaments existing at all in a patient's knee.

A top side of the cushion is attached by suitable means to the bottom of the femur, while the bottom side of the cushion is attached to the top side of the tibia, so that as the femur moves relative to the tibia, when a patient bends his or her knee joint, the positioning of the points of attachment of the cushion to the femur and the tibia are caused to shift relative to one another to accommodate the change in orientation between the femur and the tibia caused by the bending of the patient's knee joint. This shift in the relative positioning of the attachment points eliminates the sliding, and, thus, wear and tear, that occurs between the components used in conventional knee replacements where an upper metal piece of the artificial knee joint slides relative to a lower plastic piece of the joint as the joint is flexed.

The cushion can also include a valve arrangement that allows pressure to be added to or removed from the fluid filling the cushion to thereby change the level of support of a patient's body weight provided by the cushion. Where the cushion includes such a valve arrangement, a suitable pump and reservoir of fluid are implanted in the patient's leg to allow the fluid in the cushion to be adjusted and thereby change the pressure of the fluid within the cushion. Alternatively, the cushion could simply be filled using an implanted injection port.

In an alternative embodiment, two cushions can be inserted between the patient's femur and tibia. In this arrangement, the construction of each of the two cushions would be similar to that described above. However, the upper cushion would be attached on its upper side to the patient's femur, while the other, lower cushion would be attached on its lower side to the patient's tibia. Positioned between the two cushions are a pair of flat metal plates that, in one embodiment, would slide relative to one another as a patient would flex his or her knee so that the orientation of the femur relative to the tibia would change. In this arrangement, the upper cushion would be attached on its lower side to the upper metal plate, while the lower cushion would be attached on its upper side to the lower metal plate. In a further alternative embodiment, the two metal plates would be fixed relative to one another so that flexing of a patient's joint would result in shifting of the fluid within each of the two cushions so as to accommodate a change in the orientation of the patient's femur relative to the patient's tibia as the patient's knee joint is flexed.

In all of the foregoing embodiments, the cushion(s) can be inserted in a knee joint, either after the patient's menisci have been removed due to injury, or with the patient's menisci still in place, but diminished in their dimensions due to wear and tear on them.

In yet another embodiment, the flexible pocket is adapted to at least partly be attached to the tibia and femoral bones, wherein the flexible fluid filled pocket is adapted to allow a friction free movement between the tibia and femur. The movement is instead based on material flexibility and fluid movement

The cushion could also have a first fixating section for attaching the cushion to the bottom of the femur at, at least a first point of attachment and a second fixating section for attaching the cushion to the top of the tibia at at least a second point of attachment. As the femur moves relative to the tibia when a patient bends his or her knee joint, the positioning of the first and second points of attachment are caused to shift relative to one another to accommodate a change in orientation between the femur and the tibia caused by the bending of the patient's knee joint.

According to yet another embodiment, the flexible pocket in both the first and second cushion is adapted to at least partly be attached to the tibia and femoral bones. The flexible fluid filled pocket is adapted to allow friction free movement between the tibia and femur. The movement is instead based on material flexibility and fluid movement. The cushion could further comprise a first and second flat plate, positioned between the cushion and the second cushion, the first and second plates being attached to the cushion and the second cushion so that the plates slide relative to one another as the orientation between the femur and the tibia changes as the patient bends his or her knee, wherein said plates are adapted to be placed parallel to each other for allowing a larger weight carrying surface.

A method for implanting a cushion in a patient's knee joint is further described, the method comprises the steps of: placing a pocket made from a flexible material, with a fluid filling the interior of the pocket fitting in the space between the patient's femur and tibia bones.

### BRIEF DESCRIPTION OF THE DRAWINGS

FIGURE 1A is a front elevational view of a healthy knee joint.
FIGURES 1B is a top view of the medial meniscus and the lateral meniscus separating and cushioning the upper and lower knee joint bones.
FIGURE 2 is a front view of a conventional total knee replacement.
FIGURE 3 is a front view of a knee joint in an unflexed position with the cushion of the present invention implanted between a patient's femur and tibia.
FIGURES 4A to 4D are top views of several embodiments of the cushion of the present invention.

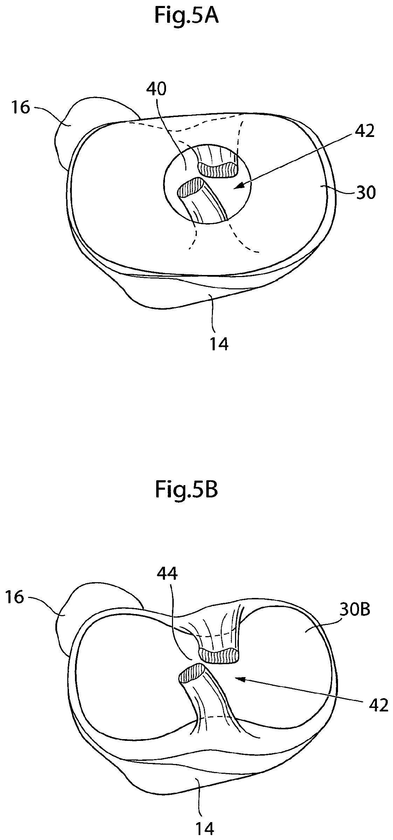

FIGURES 5A and 5B are top views of two of the embodiments of the cushion of the present invention showing their positioning on the top of a patient's tibia depicting how the cushion interacts with the two interior crucible ligaments extending from the top of the tibia to the bottom of the femur.
FIGURE 6A is a side view of a knee joint in an unflexed position with the cushion of the present invention implanted between a patient's femur and tibia.
FIGURE 6B is a side view of a knee joint in a flexed position with the cushion of the present invention implanted between a patient's femur and tibia.
FIGURE 7 is a side view of a knee joint in an unflexed position with two of the cushions of the present invention implanted between a patient's femur and tibia.
FIGURE 8 depicts one embodiment of an apparatus for performing the function of adding fluid to or removing fluid from the cushion of the present invention to thereby change the pressure in cushion
FIGURE 9 shows one embodiment of an injection port for adding fluid to or removing fluid from the cushion of the present invention.
FIGURES 10 and 11 show injection port of FIGURE 9 working as a hand driven pump.

### DETAILED DESCRIPTION OF THE INVENTION

FIGURE 3 is a front schematic view of a knee joint 10 in an unflexed position with the cushion 30 of the present invention implanted between a patient's femur 14 and tibia 12. As shown in FIGURE 3, the cushion 30 of the present invention is intended to be inserted in a patient's knee joint 10 to serve as a buffer between the patient's femur 14 and tibia 12, not unlike the menisci originally separating and cushioning the patient's femur 14 and tibia 12. As such, preferably, the cushion 30 will be sized to fit into a patient's knee joint like the original menisci 20 and 22 shown in FIGURE 1B. Preferably, the cushion 30 is filled with a substantially incompressible fluid 32, such as a foam or gel or liquid, like water, for example, which is capable of supporting the weight of a patient's body. The cushion 30 is preferably made from a flexible material 34 that allows relative movement between the patient's femur 14 and tibia 12, while supporting the weight of the patient, and while resisting rupture of the cushion 30 as it buffers the femur and tibia while they are moving relative to one another. Preferably, the cushion 30 is made from a polymer type of material. Preferably the cushion 30 is coated for increased fatigue resistance.

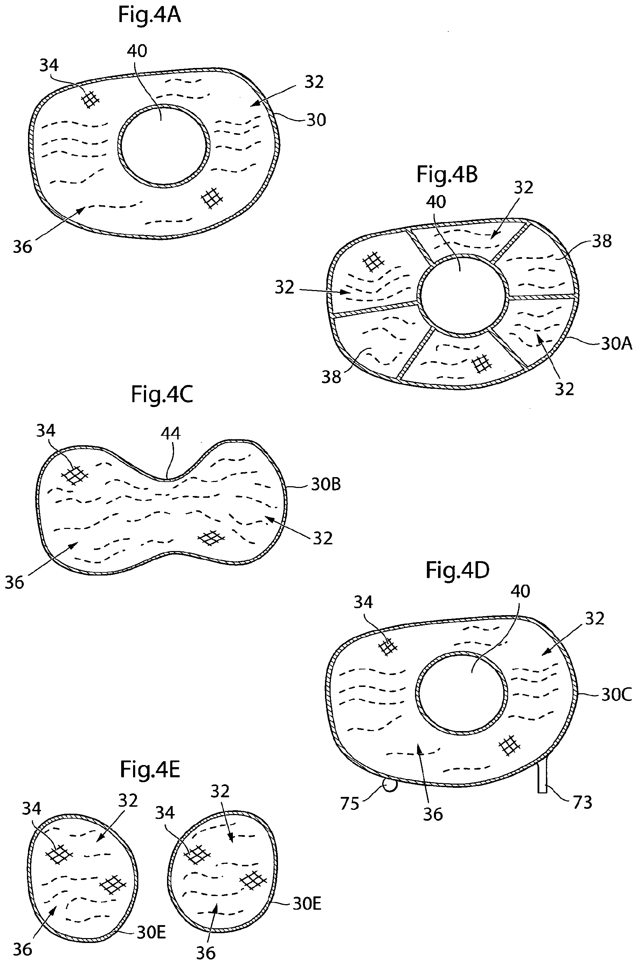

As shown in FIGURE 4A, the cushion can be formed with a single compartment 36 within which is located the substantially incompressible fluid 32. One difficulty with this design is the possibility of a complete failure of the cushion 30 where the flexible material 34 fails for some reason so that the single compartment 36 is completely ruptured, thereby allowing the fluid 32 in the compartment 36 to completely drain out of cushion 30.

FIGURE 4B is a top view of another embodiment 30A of the cushion of the present invention containing a plurality of compartments 38 within which the substantially incompressible fluid 32 is located. The cushion embodiment 30A shown in FIGURE 4B overcomes the rupturing problem of the cushion 30 of FIGURE 4A, to a degree, in that the plurality of compartments 38 within which the substantially incompressible fluid 32 is stored can prevent a complete failure of cushion 30A, if the flexible material 34 fails. In this design, if the cushion 30A is ruptured, it is likely that only a single one of the plurality of compartments 38 will rupture so as to lose the fluid 32 located in that compartment. The remaining un-ruptured compartments 38 comprising the cushion 30A will then be able to continue supporting the patient's weight and femur 14 as it is moved relative to the tibia 12 as the patient flexes his or her knee. Furthermore the compartments prevents unwanted movements of the fluid.

As shown in FIGURES 4A and 4B, each of the cushions 30 and 30A includes an opening or hole 40 in its center to accommodate the interior crucible ligaments 42 extending between the center of the bottom of the femur 14 and the top of the tibia 12. An alternative to this arrangement is the embodiment 30B of the cushion shown in FIGURE 4C. In the embodiment of FIGURE 4C, the cushion 30B is shaped like a bow tie with a narrow center portion 44 that can fit between the two interior crucible ligaments 42 extending between the femur 14 and tibia 12. It should be noted that the cushion 30B shown in FIGURE 4C could be made with plurality of compartments 38 within which the substantially incompressible fluid 32 is stored, as shown in FIGURE 4B, to again prevent a complete failure of cushion 30B where the flexible material 34 fails so as to cause only one of the plurality of compartments 38 to rupture.

The knee joint comprises a prolonged femoral length axis, being the prolongation of the length axis of the femoral bone which passes the knee joint and continues substantially to the tibia length axis, when the leg is in its fully extended state.

FIGURE 4E shows an implantable system comprising a first and second cushion according to any of the embodiments herein, the first cushion is adapted to mainly be positioned on the medial side of the femoral length axis, and the second cushion is adapted to mainly be positioned on the lateral side of the femoral length axis, such that the first cushion is adapted to mainly carry a load originating from the weight of the patient distributed through the medial condyle, and the second cushion is adapted to mainly carry a load originating from the weight of the patient distributed through the lateral condyle, when the first and second cushions are implanted.

According to another embodiment (not shown), a system could further comprise a third cushion positioned between the first cushion placed at the medial condyle and either at the femur or the tibia bones so that two cushions are positioned between the bones. The first cushion has a first fixating section for attaching the cushion to the bottom of the femur at, at least a first point of attachment, and the third cushion has a third fixating section for attaching the third cushion to the top of the tibia at, at least a third point of attachment. The system could further comprise a fourth cushion positioned between the second cushion and either the femur or the tibia bones at the lateral condyle, so that two cushions are positioned between the bones. The second cushion has a second fixating section for attaching the cushion to the bottom of the femur at, at least a second point of attachment and the fourth cushion has a fourth fixating section for attaching the fourth cushion to the top of the tibia at, at least a fourth point of attachment.

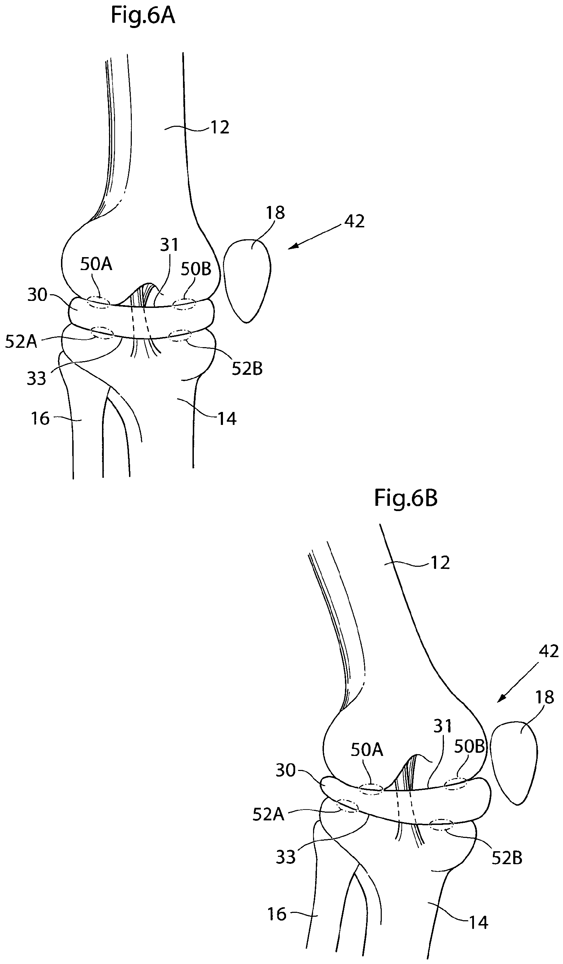

As shown in FIGURES 6A, the cushion 30 is attached by suitable means, such as by fixating sections in the form of a medical surgical adhesive or fasteners, to the femur 14 at the interface 31 of the top side of the cushion 30 and the bottom of the femur 14 at connection points 50A and 50B. Similarly, the cushion 30 is also attached by suitable means to the tibia 12 at the interface 33 of the bottom side of the cushion 30 and the top side 40 of the tibia 12 at second connection points 52A and 52B. As such, as the femur 14 moves relative to the tibia 12 when the patient bends his or her knee joint 10, as shown in FIGURE 6B, the positioning of the connection points 50A/50B and 52A/52B on the femur 14 and the tibia 12, respectively, is changed so that the connection points 50A and 50B shift relative to the connection points 52A and 52B, respectively, to accommodate a change in the orientation between the femur 14 and the tibia 12 caused by the bending of the patient's knee joint 10. This shift in the relative positioning of the attachment points 50A and 50B and 52A and 52B eliminates the sliding, and thus wear and tear, that occurs in conventional knee joint replacements, that that shown in FIGURE 2 where an upper metal piece 13 of the upper knee joint slides relative to a lower plastic piece 23 of the joint as the joint is flexed.

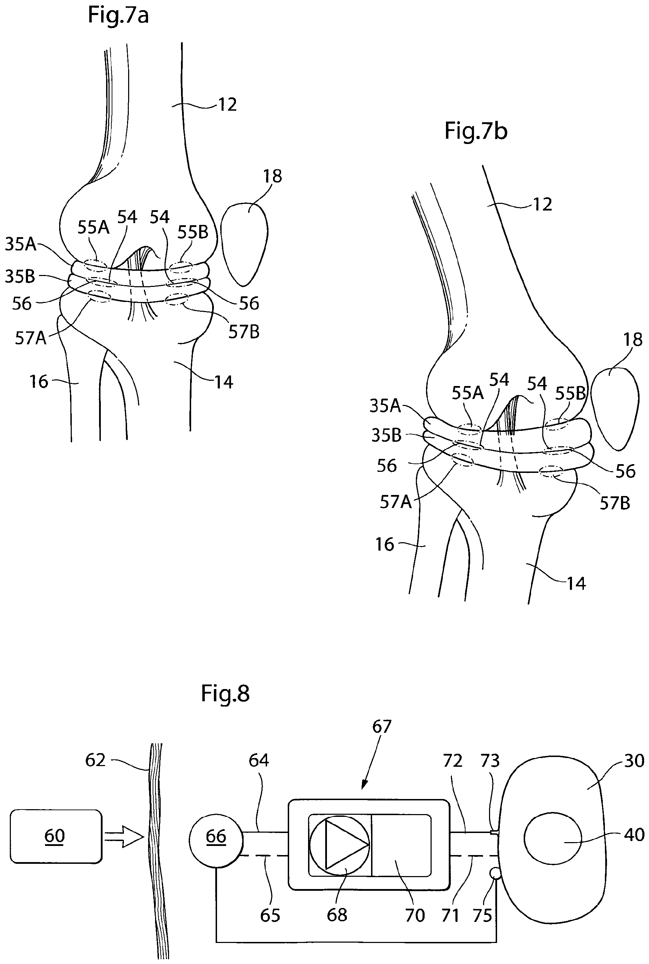

In an alternative embodiment shown in FIGURE 7A,7B, two cushions 35A and 35B can be inserted between a patient's femur and tibia. In this arrangement, the construction of each of the two cushions would be similar to that of the cushions described above and shown in FIGURES 4A to 4D. However, the upper cushion 35A would be attached on its upper side to the patient's femur 14, while the other, lower cushion 35B would be attached on its lower side to the patient's tibia 12, again, by fixating sections in the form of a suitable medical surgical adhesive or suitable medical surgical fasteners. Positioned between the two cushions are a pair of flat metal plates 54 and 56 that, in one embodiment, would slide relative to one another as a patient would flex his or her knee so that the orientation of the femur relative to the tibia would change. In this embodiment, the upper cushion 35A would be attached on its lower side to the upper metal plate 54, while the lower cushion 35B would be attached on its upper side to the lower metal plate 56. In a further alternative embodiment, the two metal plates 54 and 56 would be fixed relative to one another so that flexing of a patient's joint would result in shifting of the fluid within each of the two cushions 35A and 35 B, so as to accommodate a change in the orientation of the patient's femur relative to the patient's tibia as the patient's knee joint is flexed. FIGURE 7B shows the embodiment of FIGURE 7A when flexed.

The cushion of the present invention is constructed to allow pressure to be added to or removed from the fluid filling the cushion to thereby change the level of support of a patient's body weight provided by the cushion. As shown in FIGURES 4D, the example 30C of the cushion includes a valve arrangement 73 for adding fluid to or removing fluid from the cushion 30C and a pressure sensor 75 for determining the level of pressure in the cushion. Where the cushion includes such a valve arrangement, a suitable pump and reservoir of fluid are implanted in the patient's leg to allow the fluid in the cushion to be adjusted and thereby change the pressure of the fluid within the cushion.

FIGURE 8 depicts one embodiment of an apparatus 67 for performing the function of adding fluid to or removing fluid from the cushion 30C to thereby change the pressure in cushion 30C, whereby the cushion provides greater or lesser buffering between the knee joint bones 14 and 12. An implanted internal control unit 66 controls the pressure in the cushion 30C via control line 65. An external control unit 60, shown to the left of skin 62 in FIGURE 8, includes an external source of energy and a wireless remote control transmitting a control signal generated by the external source of energy. The control signal is received by a signal receiver incorporated in the implanted control unit 66, whereby the control unit 66 controls the pressure in the cushion 30C in response to the control signal. The implanted control unit 66 also uses energy from the control signal that is transmitted from external control unit 60 for operating, via a power supply line 64, the assembly 67 that includes a motor/pump unit 68 and a fluid reservoir 70, shown in FIGURE 8. In this case, the cushion 30C is hydraulically operated, *i.e.,* hydraulic fluid is pumped by the motor/pump unit 68 from the reservoir 70 through a conduit 72 and valve arrangement 73 to the cushion 30C to increase the pressure of the fluid 32 in the cushion 30C. Conversely, hydraulic fluid is pumped by the motor/pump unit 68 back from the cushion 30C to the reservoir 70, again through a conduit 72 and valve arrangement 73, to reduce the amount of fluid 32 and thus the pressure in the cushion 30C. The pressure is measured by pressure sensor 75, which is connected to control unit 66. The external control unit 60 releases energy carried by a wireless signal and the implanted control unit 66 transforms the wireless energy into a current, for example, for powering the motor/pump unit 68 via electric power supply line 64. The implanted control unit 66 controls the motor/pump unit 68 and the pressure in the cushion 30 via control lines 65 and 71.

The fluid supply could also only comprise an injection port that is preferably implanted subcutaneously. An injection port is disclosed in patent application publication number US 2004/0064110 A1. It should be noted that other injection port arrangements could be used with the cushion.

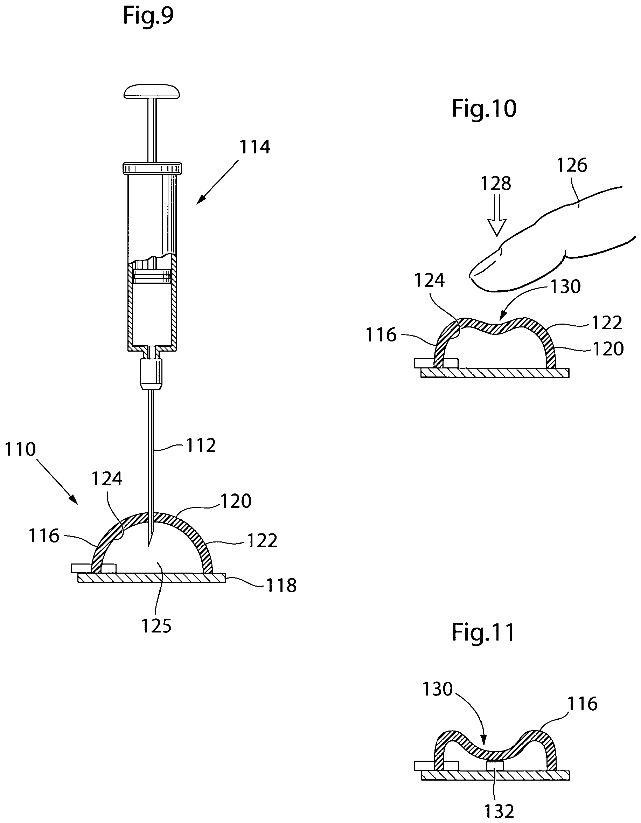

Referring to the injection port disclosed in the referenced application publication, FIGURE 9 shows an injection port 110 where a needle 112 of a syringe 114 has been injected through a membrane 116 attached to a rigid base member 18 of the injection port 110. According to the illustration shown in FIGURE 9, membrane 116 has a semi-spherical shape in its initial, "non-depressed" position, as shown in FIGURE 9. In the arrangement shown in FIGURES 9-11, membrane 116 is comprised of three layers attached to each other: a first hard layer 120 having preferably a hardness of more than 120 Shore; a second soft central layer 122 having a hardness of less than 20 Shore; and a third hard layer 124, having a hardness suitably more than 20 Shore, but preferably about 60 Shore or more. Membrane 116 and base member 118 define a chamber 125 for fluid. However, it is sufficient if membrane 116 comprises two layers, *i.e.,* one first hard layer and one second soft layer between chamber 125 and first layer 120. First layer 120 has better strength properties than second layer 122, and second layer 122 has better sealing properties than first layer 120. Membrane 116's layers are suitably made of plastic or silicone, and preferably of silicone. Suitable silicon materials are manufactured by "Applied Silicone, Inc."

FIGURES 10 and 11 show injection port 110 of FIGURE 9 working as a hand driven pump. By using a core for membrane 116 that is very soft, *i.e.*, elastic silicone material of less than 20 shore, it is possible to create a thinner and more elastic membrane that could be pumped by hand and still not cause leakage when a needle 112 of a syringe 114 penetrates the membrane. FIGURE 10 illustrates a finger 126 pushing (actuated by one push) membrane 116 in a direction 128 from above. Membrane 116 will then be substantially flattened, such that the surface that is faced against the finger 126 will assume a somewhat concave bowl-shape 130. Membrane 116 is then moved to a lowest position, as shown in FIGURE 11, where it is held by a locking device 132 until it is manually pressed again. When membrane 116 is actuated again, by a second push by the finger 126, the locking device 132 (which functions similar to the locking mechanism for a ballpoint pen) releases membrane 116, whereby membrane 116 is able to return to its regular convex-shaped condition as shown in FIGURE 9.

According to the method of using injection port 110 to add fluid to cushion 130, after injection port 110 is subcutaneously implanted in the patient, displaceable injection membrane 116 is used to pump fluid in fluid chamber 125 to cushion 130, which is hydraulically connected to injection port 110. The amount of fluid in fluid chamber 125 capable of being pumped to restriction device 141 using injection port 110 is calibrated by penetrating the patient's skin and membrane 116 of injection port 110 with injection needle 112 of syringe 114 to add or withdraw fluid from chamber 125. Membrane 116 is manually displaced from time to time to pump the fluid from chamber 125 of injection port 110 to implant 141 to operate the implant.

While the invention has been described in connection with what is presently considered to be the most practical and preferred embodiment, it is to be understood that the invention is not to be limited to the disclosed embodiment, but on the contrary, is intended to cover various modifications within the scope of the invention as defined by the appended claims.

## Claims

1. A cushion adapted to be implanted in a patient's knee joint between the patient's femur and tibia bones, the cushion (30C, 35A, 35B) comprising:
- a pocket made from a flexible material, and a fluid (32) filling the interior of the pocket,
- the pocket being sized to fit in the space between the patient's femur and tibia bones,
**characterized in that** the cushion further comprises
- an internal control unit (66) adapted to be implanted and control the pressure in the implanted cushion (30C, 35A, 35B), non-invasively when implanted,
wherein the cushion (30C, 35A, 35B), when implanted is adapted to allow pressure to be added to or removed from the fluid filling the cushion (30C, 35A, 35B) non-invasively, and
wherein the cushion further comprises a pressure sensor (75) connected to the cushion and to the implanted internal control unit (66), the pressure sensor (75) being configured to, postoperatively when implanted, determine the level of pressure in the cushion.

2. The cushion of claim 1, wherein the pocket is adapted to accommodate two interior crucible ligaments extending between the patient's femur and tibia bones.

3. The cushion according to any one of claims 1-2, wherein the cushion substantially has a size corresponding to the menisci originally in the patient's knee joint between the patient's femur and tibia bones.

4. The cushion according to any one of claims 1-3, wherein the fluid is at least one of;
a substantially incompressible fluid,
water being a substantially incompressible fluid,
a foam,
a gel, and
adapted to be included in the cushion of flexible material being a polymer type of material.

5. The cushion according to any one of claims 1-4, wherein the cushion is comprised of a plurality of interior pockets filled with the fluid and isolated from another so that a rupture of one pocket results in the fluid draining from the ruptured pocket and not from the other pockets comprising the plurality of pockets.

6. The cushion according to any one of claims 1-5, wherein the cushion has a first fixating section for attaching the cushion to the bottom of the femur at, at least a first point of attachment and a second fixating section for attaching the cushion to the top of the tibia at, at least a second point of attachment so that, as the femur moves relative to the tibia when a patient bends his or her knee joint, the positioning of the first and second points of attachment are caused to shift relative to one another to accommodate a change in orientation between the femur and the tibia caused by the bending of the patient's knee joint.

7. The cushion according to any one of claims 1-6, further comprising an apparatus adapted to be implanted in a patient that is connected to the valve arrangement for adding fluid to, or removing fluid from, the cushion, wherein the apparatus is comprised of: a fluid reservoir adapted to be implanted in the patient from which fluid is obtained or to which fluid is added for changing the level of fluid in the cushion, and
a motor pump unit adapted to be implanted in a patient for transferring fluid from the fluid reservoir to the cushion and from the cushion to the fluid reservoir to thereby change the pressure in the cushion,
wherein the apparatus further comprising;
an external control unit including an external source of energy, and a wireless remote control transmitting to the internal control unit a control signal generated by the external source of energy.

8. The cushion according to any one of claims 1-7, further comprising a second cushion positioned between the cushion and either the femur or the tibia bones so that two cushions are positioned between said bones, wherein the cushion has a first fixating section for attaching the cushion to the bottom of the femur at least a first point of attachment and the second cushion has a second fixating section for attaching the second cushion to the top of the tibia at least a second point of attachment.

9. The cushion of claim 8, further comprising first and second flat metal plates positioned between the cushion and the second cushion, the first and second metal plates being attached to the cushion and the second cushion so that the plates are adapted to at least one of;
slide relative to one another as the orientation between the femur and the tibia changes as the patient bends his or her knee, and
being fixed relative to one another so that the first and second points of attachment are caused to shift relative to one another to accommodate a change in orientation between the femur and the tibia caused by the bending of the patient's knee joint.

10. The cushion of any one of claims 7-9, wherein the pressure sensor supplies to the internal control unit a signal representative of the level of pressure in the cushion, wherein the apparatus adds fluid to, or removes fluid from, the cushion, depending on the level of pressure in the cushion measured by the pressure sensor connected to the cushion.

11. The cushion according to any one of claims 1-10, further comprising an implanted injection port for adding fluid to, or removing fluid from, the cushion.

12. A system adapted to be implanted in a patient's knee joint, the knee joint comprising a prolonged femoral length axis, being the prolongation of the length axis of the femoral bone which passes the knee joint and continues substantially to the tibia length axis, when the leg is in its fully extended state, wherein said system comprises:
a first cushion according to claim 1,
and
a second cushion according to claim 1,
wherein:
said first cushion is adapted to mainly be positioned on the medial side of said femoral length axis,
said second cushion is adapted to mainly be positioned on the lateral side of said femoral length axis, such that:
said first cushion is adapted to mainly carry a load originating from the weight of the patient distributed through the medial condyle, and
said second cushion is adapted to mainly carry a load originating from the weight of the patient distributed through the lateral condyle, when said first and second cushions are implanted.

13. The system of claim 12, further comprising a third cushion positioned between the first cushion placed at the medial condyle and either at the femur or the tibia bones so that two cushions are positioned between the bones, wherein said first cushion has a first fixating section for attaching the cushion to the bottom of the femur at, at least a first point of attachment, and the third cushion has a third fixating section for attaching the third cushion to the top of the tibia at, at least a third point of attachment,
wherein the system further comprising a fourth cushion positioned between the second cushion and either the femur or the tibia bones at the lateral condyle so that two cushions are positioned between said bones, wherein the second cushion has a second fixating section for attaching the cushion to the bottom of the femur at, at least a second point of attachment and the fourth cushion has a fourth fixating section for attaching the fourth cushion to the top of the tibia at, at least a fourth point of attachment.

14. The cushion according to claim 1, wherein the flexible pocket is adapted to at least partly be attached to the tibia and femoral bones, wherein the flexible fluid filled pocket is adapted to allow a friction free movement between the tibia and femur, said movement instead based on material flexibility and fluid movement

15. The cushion of claim 14, wherein the cushion has a first fixating section for attaching the cushion to the bottom of the femur at, at least a first point of attachment and a second fixating section for attaching
the cushion to the top of the tibia at, at least a second point of attachment so that, as the femur moves relative to the tibia when a patient bends his or her knee joint, the positioning of the first and second points of attachment are caused to shift relative to one another to accommodate a change in orientation between the femur and the tibia caused by the bending of the patient's knee joint.

## Patentansprüche

1. Polster, das dazu ausgeführt ist, in ein Kniegelenk eines Patienten zwischen dem Femur- und dem Tibiaknochen eines Patienten implantiert zu werden, wobei das Polster (30C, 35A, 35B) Folgendes umfasst:
- eine aus einem flexiblen Material hergestellte Tasche und ein das Innere der Tasche füllendes Fluid (32),
- wobei die Tasche so bemessen ist, dass sie in den Raum zwischen dem Femur- und dem Tibiaknochen des Patienten passt,
**dadurch gekennzeichnet, dass** das Polster ferner Folgendes umfasst:
- eine innere Steuereinheit (66), die dazu ausgeführt ist, implantiert zu werden und den Druck in dem implantierten Polster (30C, 35A, 35B) nicht invasiv nach der Implantation zu steuern,
wobei das Polster (30C, 35A, 35B) nach der Implantation dazu ausgeführt ist, zu gestatten, dass der Druck auf das das Polster (30C, 35A, 35B) füllende Fluid nicht invasiv erhöht oder entlastet wird, und wobei das Polster ferner einen Drucksensor (75) umfasst, der mit dem Polster und mit der implantierten inneren Steuereinheit (66) verbunden ist, wobei der Drucksensor (75) dazu ausgestaltet ist, postoperativ nach der Implantation das Druckniveau in Polster zu bestimmen.

2. Polster nach Anspruch 1, wobei die Tasche dazu ausgeführt ist, zwei sich zwischen dem Femur- und dem Tibiaknochen des Patienten erstreckende innere Kreuzbänder aufzunehmen.

3. Polster nach einem der Ansprüche 1 - 2, wobei das Polster im Wesentlichen eine Größe hat, die den sich ursprünglich in dem Kniegelenk des Patienten zwischen dem Femur- und dem Tibiaknochen des Patienten befindenden Menisken entspricht.

4. Polster nach einem der Ansprüche 1 - 3, wobei das Fluid
ein im Wesentlichen inkompressibles Fluid,
wobei Wasser ein im Wesentlichen inkompressibles Fluid ist, und/oder
ein Schaum und/oder
ein Gel ist und
dazu ausgeführt ist, in dem Polster aus flexiblem Material, bei dem es sich um ein polymerartiges Material handelt, aufgenommen zu sein.

5. Polster nach einem der Ansprüche 1 - 4, wobei das Polster aus einer Vielzahl von inneren Taschen besteht, die mit dem Fluid gefüllt und von einander isoliert sind, so dass ein Riss einer Tasche dazu führt, dass das Fluid aus der gerissenen Tasche ausläuft und nicht aus den anderen Taschen, die die Vielzahl von Taschen umfassen.

6. Polster nach einem der Ansprüche 1 - 5, wobei das Polster einen ersten Fixierungsabschnitt zum Anbringen des Polsters an dem unteren Teil des Femurs an mindestens einer ersten Anbringstelle und einen zweiten Fixierungsabschnitt zum Anbringen des Polsters an dem oberen Teil der Tibia an mindestens einer zweiten Anbringstelle hat, so dass veranlasst wird, dass sich die Positionierung der ersten und die Positionierung der zweiten Anbringstelle bezüglich einander verschieben, wenn sich das Femur bezüglich der Tibia bewegt, wenn ein Patient sein Kniegelenk beugt, um einer durch das Beugen des Kniegelenks des Patienten veranlassten Ausrichtungsänderung zwischen dem Femur und der Tibia Rechnung zu tragen.

7. Polster nach einem der Ansprüche 1 - 6, ferner umfassend eine zur Implantierung in einen Patienten ausgeführte Vorrichtung, die mit der Ventilanordnung verbunden ist, um dem Polster Fluid hinzuzufügen oder Fluid daraus zu entfernen, wobei die Vorrichtung aus Folgendem besteht: einem zur Implantation in den Patienten ausgeführten Fluidreservoir, aus dem Fluid erhalten wird oder zu dem Fluid hinzugefügt wird, um den Fluidpegel in dem Polster zu ändern, und
eine zur Implantation in einen Patienten ausgeführte Motorpumpeneinheit zum Transferieren von Fluid aus dem Fluidreservoir zu dem Polster und von dem Polster zu dem Fluidreservoir, um dadurch den Druck in dem Polster zu ändern,
wobei die Vorrichtung ferner Folgendes umfasst:
eine äußere Steuereinheit, die eine äußere Energiequelle aufweist, und eine drahtlose Fernsteuerung, die ein von der äußeren Energiequelle generiertes Steuersignal an die innere Steuereinheit sendet.

8. Polster nach einem der Ansprüche 1 - 7, ferner umfassend ein zweites Polster, das zwischen dem Polster und entweder dem Femur- oder dem Tibiaknochen positioniert ist, so dass zwei Polster zwischen den Knochen positioniert sind, wobei das Polster einen ersten Fixierungsabschnitt zum Anbringen des Polsters an dem unteren Teil des Femurs an mindestens einer ersten Anbringstelle hat und das zweite Polster einen zweiten Fixierungsabschnitt zum Anbringen des zweiten Polsters an dem oberen Teil der Tibia an mindestens einer zweiten Anbringstelle hat.

9. Polster nach Anspruch 8, ferner umfassend eine erste und eine zweite flache Metallplatte, die zwischen dem Polster und dem zweiten Polster positioniert sind, wobei die erste und die zweite Metallplatte an dem Polster und dem zweiten Polster angebracht sind, so dass die Platten dazu ausgeführt sind:
bezüglich einander zu gleiten, wenn sich die Ausrichtung zwischen dem Femur und der Tibia ändert, wenn der Patient sein Knie beugt, und/oder bezüglich einander fixiert zu sein, so dass veranlasst wird, dass sich die erste und die zweite Anbringstelle bezüglich einander verschieben, um einer durch das Beugen des Kniegelenks des Patienten veranlassten Ausrichtungsänderung zwischen dem Femur und der Tibia Rechnung zu tragen.

10. Polster nach einem der Ansprüche 7 - 9, wobei der Drucksensor der inneren Steuereinheit ein Signal liefert, das für das Druckniveau in dem Polster repräsentativ ist, wobei die Vorrichtung dem Polster Fluid hinzufügt oder Fluid daraus entfernt, je nach dem Druckniveau in dem Polster, das mittels des mit dem Polster verbundenen Drucksensors gemessen wird.

11. Polster nach einem der Ansprüche 1 - 10, ferner umfassend einen implantierten Injektionsport zum Hinzufügen von Fluid zu dem Polster oder zum Entfernen von Fluid daraus.

12. System, das zur Implantation in einem Kniegelenk eines Patienten ausgeführt ist, wobei das Kniegelenk eine verlängerte Femurlängsachse umfasst, bei der es sich um die Verlängerung der Längsachse des Femurknochens handelt, die an dem Kniegelenk vorbeiführt und im Wesentlichen zu der Tibialängsachse weiterläuft, wenn das Bein in seinem vollständig gestreckten Zustand ist, wobei das System Folgendes umfasst:
ein erstes Polster nach Anspruch 1
und
ein zweites Polster nach Anspruch 1,
wobei:
das erste Polster dazu ausgeführt ist, hauptsächlich an der medialen Seite der Femurlängsachse positioniert zu sein,
das zweite Polster dazu ausgeführt ist, hauptsächlich an der lateralen Seite der Femurlängsachse positioniert zu sein, so dass:
das erste Polster dazu ausgeführt ist, in der Hauptsache eine von dem durch den Condylus medialis verteilten Gewicht des Patienten ausgehende Last zu tragen, und
das zweite Polster dazu ausgeführt ist, in der Hauptsache eine von dem durch den Condylus lateralis verteilten Gewicht des Patienten ausgehende Last zu tragen, wenn das erste und das zweite Polster implantiert sind.

13. System nach Anspruch 12, ferner umfassend ein drittes Polster, das zwischen dem ersten Polster, das an dem Condylus medialis platziert ist, und entweder an dem Femur- oder dem Tibiaknochen positioniert ist, so dass zwei Polster zwischen den Knochen positioniert sind, wobei das erste Polster einen ersten Fixierungsabschnitt zum Anbringen des Polsters an dem unteren Teil des Femurs an mindestens einer ersten Anbringstelle hat und das dritte Polster einen dritten Fixierungsabschnitt zum Anbringen des dritten Polsters an dem oberen Teil der Tibia an mindestens einer dritten Anbringstelle hat, wobei das System ferner ein viertes Polster umfasst, das zwischen dem zweiten Polster und entweder dem Femur- oder dem Tibiaknochen an dem Condylus lateralis positioniert ist, so dass zwei Polster zwischen den Knochen positioniert sind, wobei das zweite Polster einen zweiten Fixierungsabschnitt zum Anbringen des Polsters an dem unteren Teil des Femurs an mindestens einer zweiten Anbringstelle hat und das vierte Polster einen vierten Fixierungsabschnitt zum Anbringen des vierten Polsters an dem oberen Teil der Tibia an mindestens einer vierten Anbringstelle hat.

14. Polster nach Anspruch 1, wobei die flexible Tasche dazu ausgeführt ist, mindestens teilweise an dem Femur- und dem Tibiaknochen angebracht zu werden, wobei die flexible, mit Fluid gefüllte Tasche dazu ausgeführt ist, eine reibungslose Bewegung zwischen der Tibia und dem Femur zu gestatten, wobei die Bewegung stattdessen auf Materialflexibilität und Fluidbewegung beruht.

15. Polster nach Anspruch 14, wobei das Polster einen ersten Fixierungsabschnitt zum Anbringen des Polsters an dem unteren Teil des Femurs an mindestens einer ersten Anbringstelle und einen zweiten Fixierungsabschnitt zum Anbringen des Polsters an dem oberen Teil der Tibia an mindestens einer zweiten Anbringstelle hat, so dass veranlasst wird, dass sich die Positionierung der ersten und die Positionierung der zweiten Anbringstelle bezüglich einander verschieben, wenn sich das Femur bezüglich der Tibia bewegt, wenn ein Patient sein Kniegelenk beugt, um einer durch das Beugen des Kniegelenks des Patienten veranlassten Ausrichtungsänderung zwischen dem Femur und der Tibia Rechnung zu tragen.

## Revendications

1. Coussin prévu pour être implanté dans une articulation du genou d'un patient entre le fémur et le tibia du patient, le coussin (30C, 35A, 35B) comprenant :
- une poche fabriquée en matériau souple, et un fluide (32) remplissant l'intérieur de la poche,
- la poche étant dimensionnée pour s'ajuster dans l'espace entre le fémur et le tibia du patient, **caractérisé en ce que** le coussin comprend en outre
- une unité de commande interne (66) prévue pour être implantée et pour réguler la pression dans le coussin implanté (30C, 35A, 35B) de manière non invasive lorsqu'il est implanté,
le coussin (30C, 35A, 35B), lorsqu'il est implanté, étant prévu pour permettre d'augmenter ou de réduire la pression dans le fluide remplissant le coussin (30C, 35A, 35B) de manière non invasive, et
le coussin comprenant en outre un capteur de pression (75) connecté au coussin et à l'unité de commande interne implantée (66), le capteur de pression (75) étant configuré, après l'intervention, lorsqu'il est implanté, pour déterminer le niveau de pression dans le coussin.

2. Coussin selon la revendication 1, dans lequel la poche est prévue pour recevoir deux ligaments croisés intérieurs s'étendant entre le fémur et le tibia du patient.

3. Coussin selon l'une quelconque des revendications 1 et 2, le coussin ayant sensiblement une taille correspondant au ménisque d'origine dans l'articulation du genou du patient entre le fémur et le tibia du patient.

4. Coussin selon l'une quelconque des revendications 1 à 3, dans lequel le fluide est au moins l'un parmi :
un fluide substantiellement incompressible,
de l'eau, qui est un fluide substantiellement incompressible,
une mousse,
un gel, et
prévu pour être inclus dans le coussin en matériau souple qui est un type de matériau polymère.

5. Coussin selon l'une quelconque des revendications 1 à 4, le coussin étant constitué d'une pluralité de poches intérieures remplies avec le fluide et isolées les unes des autres de telle sorte qu'une rupture d'une poche entraîne le drainage du fluide de la poche rompue et non pas des autres poches constituant la pluralité de poches.

6. Coussin selon l'une quelconque des revendications 1 à 5, le coussin ayant une première section de fixation pour attacher le coussin à la base du fémur au niveau d'au moins un premier point d'attache et une deuxième section de fixation pour attacher le coussin à la partie supérieure du tibia au niveau d'au moins un deuxième point d'attache de telle sorte qu'à mesure que le fémur se déplace par rapport au tibia lorsqu'un patient fléchit son articulation de genou, le positionnement des premier et deuxième points d'attache soient amenés à se déplacer l'un par rapport à l'autre pour accommoder un changement d'orientation entre le fémur et le tibia provoqué par la flexion de l'articulation du genou du patient.

7. Coussin selon l'une quelconque des revendications 1 à 6, comprenant en outre un appareil prévu pour être implanté dans un patient, lequel est connecté à l'agencement de valve pour ajouter du fluide au coussin ou retirer du fluide de celui-ci, l'appareil étant constitué :
d'un réservoir de fluide prévu pour être implanté dans le patient, depuis lequel du fluide est obtenu ou dans lequel du fluide est ajouté pour faire varier le niveau de fluide dans le coussin, et
d'une unité de pompe à moteur prévue pour être implantée dans un patient pour transférer du fluide depuis le réservoir de fluide jusqu'au coussin et depuis le coussin jusqu'au réservoir de fluide pour ainsi faire varier la pression dans le coussin,
l'appareil comprenant en outre :
une unité de commande externe comportant une source d'énergie externe, et une commande à distance sans fil transmettant à l'unité de commande interne un signal de commande généré par la source d'énergie externe.

8. Coussin selon l'une quelconque des revendications 1 à 7, comprenant en outre un deuxième coussin positionné entre le coussin et soit le fémur soit le tibia de telle sorte que deux coussins soient positionnés entre lesdits os, le coussin ayant une première section de fixation pour attacher le coussin à la base du fémur au niveau d'au moins un premier point d'attache et le deuxième coussin ayant une deuxième section de fixation pour attacher le deuxième coussin à la partie supérieure du tibia au niveau d'au moins un deuxième point d'attache.

9. Coussin selon la revendication 8, comprenant en outre des première et deuxième plaques métalliques plates positionnées entre le coussin et le deuxième coussin, les première et deuxième plaques métalliques étant attachées au coussin et au deuxième coussin de telle sorte que les plaques prévues pour :
coulisser l'une par rapport à l'autre à mesure que l'orientation entre le fémur et le tibia varie à mesure que le patient fléchit son genou, et/ou être fixées l'une par rapport à l'autre de telle sorte que les premier et deuxième points d'attache soient amenés à se déplacer l'un par rapport à l'autre pour accommoder un changement d'orientation entre le fémur et le tibia provoqué par la flexion de l'articulation du genou du patient.

10. Coussin selon l'une quelconque des revendications 7 à 9, dans lequel le capteur de pression fournit à l'unité de commande un signal représentatif du niveau de pression dans le coussin, l'appareil ajoutant du fluide au coussin ou retirant du fluide du coussin en fonction du niveau de pression dans le coussin, mesuré par le capteur de pression connecté au coussin.

11. Coussin selon l'une quelconque des revendications 1 à 10, comprenant en outre un orifice d'injection implanté pour ajouter du fluide au coussin ou pour retirer du fluide du coussin.

12. Système prévu pour être implanté dans une articulation du genou d'un patient, l'articulation du genou comprenant un axe longitudinal fémoral prolongé, constituant le prolongement de l'axe longitudinal du fémur qui passe par l'articulation du genou et continue substantiellement jusqu'à l'axe longitudinal du tibia lorsque la jambe est dans son état complètement étendu, ledit système comprenant :
un premier coussin selon la revendication 1, et
un deuxième coussin selon la revendication 1,
dans lequel
ledit premier coussin est prévu pour être positionné principalement sur le côté médial dudit axe longitudinal fémoral,
ledit deuxième coussin est prévu pour être positionné principalement sur le côté latéral dudit axe longitudinal fémoral, de telle sorte que :
ledit premier coussin soit prévu pour supporter principalement une charge résultant du poids du patient réparti sur le condyle médial et
ledit deuxième coussin soit prévu pour supporter principalement une charge résultant du poids du patient réparti sur le condyle latéral, lorsque lesdits premier et deuxième coussins sont implantés.

13. Système selon la revendication 12, comprenant en outre un troisième coussin positionné entre le premier coussin placé au niveau du condyle médial et au niveau soit du fémur soit du tibia de telle sorte que deux coussins soient positionnés entre les os, ledit premier coussin ayant une première section de fixation pour attacher le coussin à la partie inférieure du fémur au niveau d'au moins un premier point d'attache, et le troisième coussin ayant une troisième section de fixation pour attacher le troisième coussin à la partie supérieure du tibia au niveau d'au moins un troisième point d'attache,
le système comprenant en outre un quatrième coussin positionné entre le deuxième coussin et soit le fémur soit le tibia au niveau du condyle latéral de telle sorte que deux coussins soient positionnés entre lesdits os, le deuxième coussin ayant une deuxième section de fixation pour attacher le coussin à la partie inférieure du fémur au niveau d'au moins un deuxième point d'attache et le quatrième coussin ayant une quatrième section de fixation pour attacher le quatrième coussin à la partie supérieure du tibia au niveau d'au moins un quatrième point d'attache.

14. Coussin selon la revendication 1, dans lequel la poche souple est prévue pour être attachée au moins en partie au tibia et au fémur, la poche souple remplie de fluide étant prévue pour permettre un mouvement sans frottement entre le tibia et le fémur, ledit mouvement étant au contraire basé sur la souplesse du matériau et le mouvement du fluide.

15. Coussin selon la revendication 14, le coussin ayant une première section de fixation pour attacher le coussin à la partie inférieure du fémur au niveau d'au moins un premier point d'attache et une deuxième section de fixation pour attacher le coussin à la partie supérieure du tibia au niveau d'au moins un deuxième point d'attache de telle sorte qu'à mesure que le fémur se déplace par rapport au tibia lorsqu'un patient fléchit son articulation de genou, le positionnement des premier et deuxième points d'attache soient amenés à se déplacer l'un par rapport à l'autre pour accommoder un changement d'orientation entre le fémur et le tibia provoqué par la flexion de l'articulation du genou du patient.
